# EUROPEAN PATENT APPLICATION

(11) **EP 3 984 440 A1**
(43) Date of publication of application: **20.04.2022**
(21) Application number: 19932846.9
(22) Date of filing: 13.06.2019
(51) Int. Cl.: A61B 1/04

(54) **ENDOTRACHEAL TUBE APPLIED TO BUILT-IN VIDEO ENDOSCOPE AND CAPABLE OF FULL-TIME VIDEO MONITORING**

(71) Applicant: Chang, Li Way, New Taipei City, Taiwan (TW)
(72) Inventor: Chang, Li Way, New Taipei City, Taiwan (TW)
(74) Representative: Zimmermann, Tankred Klaus
(86) International application number: PCT/CN2019/091014
(87) International publication number: WO 2020/248180

(57) **Abstract**

An endotracheal tube applied to a built-in video endoscope and capable of full-time video monitoring, which comprises a tubular body (10) and optical cleaning rods (20). The tubular body (10) is to be placed in the trachea (2) of a patient (1); a front end (11) of the tubular body (10) is connected to a ventilator, and an inflatable bag (13) is provided in the tubular body (10); a primary channel (14) of the tubular body (10) runs through the front and rear ends (11, 12), so that the ventilator supplies oxygen to the trachea (2) of the patient (1) from the primary channel (14); each secondary channel (15) of the tubular body (10) is provided with a front opening (151) and a rear opening (152), the front opening (151) is located at a side edge of the tubular body (10), the rear opening (152) is arranged at the rear end (12), and when the tubular body (10) is placed in the trachea (2) of the patient (1), the front opening (151) is positioned higher than a bite part (3) of the patient (1); the optical cleaning rods (20) run through the secondary channels (15), each optical cleaning rod (20) is provided with a camera device (23) coupled to a display, and the camera device (23) comes out from the rear opening (152) to capture a video in the trachea (2) of the patient (1).

## Description

### BACKGROUND

### Technology Field

The present disclosure relates to an endotracheal tube and, in particular, to an endotracheal tube with built-in video endoscope function that can be applied to routine and difficult endotracheal intubation and have built-in video endoscope function for full-time video monitoring, so that it can full-time monitoring the position of the endotracheal tube, the internal structure of trachea and various conditions.

### Description of Related Art

Endotracheal intubation is used for airway management and definite airway, and is an important first aid in the medical fields of the first-line emergency, trauma, and critical care. Patients may have respiratory failure caused by pneumonia or acute pulmonary edema, coma caused by stroke or hypoglycemia, upper respiratory tract obstruction caused by facial trauma endangering the respiratory tract, even by acute tonsillitis or foreign body choking, and require emergency intervention to maintain the oxygenation and ventilation of the patient and to avoid irreversible complications caused by hypoxia.

Endotracheal intubation is to insert an endotracheal tube into the trachea of a patient and to connect the endotracheal tube to a ventilator so that the ventilator can deliver oxygen to the trachea of the patient. The endotracheal intubation can ensure that the patient's respiratory tract is unblocked and prevent foreign objects from being inhaled into the lungs, so as to ensure that the patient has adequate ventilation.

When performing endotracheal intubation, the operator must ensure that the endotracheal tube is correctly inserted into the trachea and avoid inserting the endotracheal tube into the esophagus that is very close to the trachea. The current medical methods to ensure the correct intubation position and depth of the endotracheal tube include auscultation with a stethoscope, checking whether there is any fogging on the wall of the endotracheal tube, taking X-rays after intubation, and using end-tidal carbon dioxide (EtCO₂) monitor. EtCO₂ is detected by a carbon dioxide detector disposed between the endotracheal tube and the ventilator. If the concentration of the carbon dioxide in the patent's exhalation is high, it can be confirmed that the endotracheal tube is correctly inserted into the trachea. However, the said EtCO₂ monitor has some defects, such as the carbon dioxide detector is easily blocked by the patient's sputum or secretions, which may affect detection results. Moreover, it is easy to cause inaccurate detection or detection errors of the EtCO₂ monitor when the patient is moved or shaken, such as in the situations that the patient is transported to ICU or transported in the emergency ambulance. In addition, there is a risk that the endotracheal tube could be pulled out easily since the EtCO₂ monitor is very heavy.

Because of the said defects, it is needed to develop another method that is more stable and more effective to detect and ensure the position of the endotracheal tube. A conventional optical-stylet endotracheal intubation is to assemble an optical stylet with a camera at one end into the center channel of the endotracheal tube and then insert the optical stylet together with the endotracheal tube into the trachea of a patient. Therefore, if the carina of the patient can be observed on a display connecting to the camera, the intubation position of the endotracheal tube is correct. Then, the optical stylet should be removed before the endotracheal tube is connected to the ventilator.

At the time of performing the endotracheal intubation, the position of the endotracheal tube can be confirmed by observing the carina of the patient, and then the position of the endotracheal tube is reconfirmed by several methods such as auscultation, inspection of the fogging on the wall of the endotracheal tube, taking X-rays after intubation, and end-tidal carbon dioxide (EtCO₂) monitor. However, the position of the endotracheal tube could be changed by the patient's transportation, moving, turning over, or expectoration. Moreover, many conditions will cause the risk of slippage and dislocation of the endotracheal tube when the patient is transported into ICU from emergency rooms, transported out of the ICU for any detections, and transferred to another hospital, coughing or performing sputum suction of the intubated patient, turning over the anesthetized patient in the operation room for the surgery. When any of the previous situations happens, the incorrect position of the endotracheal tube is often observed too late to cause the tragedy in airway management.

### SUMMARY

An objective of this disclosure is to provide an endotracheal tube with a built-in video endoscope function for full-time video monitoring, which has a primary channel for a ventilator to deliver oxygen to a patient's trachea, and a secondary channel for an optical stylet with a camera device to pass through, thereby achieving the purpose of full-time monitoring the position of the endotracheal tube, the internal structure of trachea and various conditions.

The technical solution to solve the above problem is implemented as follows:

One or more exemplary embodiment of this present disclosure provides an endotracheal tube with built-in video endoscope function for full-time video monitoring, which comprises: a tubular body being flexible and having a pre-shaped curvature, wherein the tubular body is to be placed in a trachea of a patient, the tubular body has a front end and a rear end opposite to each other, the front end is connected to a ventilator, the tubular body is configured with an inflatable bag, a primary channel and a secondary channel run through the tubular body, the primary channel runs through the front end and the rear end, so that the ventilator supplies oxygen to the trachea of the patient through the primary channel, the secondary channel is located next to the primary channel and is provided with a front opening and a rear opening communicating with each other, the front opening is located at a side edge of the tubular body, the rear opening is arranged at the rear end, and when the tubular body is placed in the trachea of the patient, the front opening is positioned higher than a bite part of the patient; and an optical stylet having a length greater than that of the tubular body and being flexible, wherein the optical stylet runs through the front opening and the rear opening of the secondary channel, an end of the optical stylet is provided with a camera device, and the camera device is coupled to a display, and the end of the optical stylet comes out from the rear opening to capture a video in the trachea of the patient.

In one exemplary embodiment, when the end of the optical stylet is located at the rear opening, a part of the optical stylet coming out from the front opening has a measuring scale.

In one exemplary embodiment, the rear end of the tubular body has an oblique opening structure, and an oblique angle of the rear end of the tubular body is greater than 0 and less than 90 degrees.

In one exemplary embodiment, the endotracheal tube comprises a plurality of the secondary channels, and the optical stylet runs through one of the secondary channels.

In one exemplary embodiment, the secondary channels are arranged with equivalent spaced angle.

In one exemplary embodiment, the optical stylet comes out from the rear opening of the corresponding secondary channel, and the camera devices aim to different directions to capture the video.

In one exemplary embodiment, the side edge of the tubular body is formed with a Murphy eye, and the Murphy eye is close to the rear end and communicates with the primary channel.

In one exemplary embodiment, an aperture size of the Murry eye is equal to an inner diameter of the primary channel, or the aperture size of the Murry eye is smaller than the inner diameter of the primary channel and larger than an inner diameter of the secondary channel.

In one exemplary embodiment, a radial cross-section of the tubular body is substantially elliptical, and a radial cross-section of the primary channel is substantially elliptical.

In one exemplary embodiment, the camera device is remotely coupled to the display.

According to the above technical solution, the present disclosure can achieve the effect of: the oxygen provided by the ventilator and the optical stylet can enter the tubular body through the primary channel and the secondary channel respectively. Therefore, during the intubation of the endotracheal tube, the optical stylet protrudes from the tubular body for leading and guiding the tubular body, and through the camera device at the end of the optical stylet and the display connected to the camera device, the operator can continuously monitor the intubation process of the endotracheal tube at all times, and identify and find the glottis (i.e., the entrance of trachea) at the front end. At the same time, different from the intubation of conventional endotracheal tube and optical stylet, the built-in optical stylet can be pulled back and retracted to even with the end of the tubular body after the intubation of endotracheal tube is completed. In this case, the endotracheal tube is used as the full-time monitor for monitoring the internal conditions of the trachea, bleeding or expectoration, and the position of the endotracheal tube. Compared to the conventional optical stylet, which must be removed after the intubation of endotracheal tube (so it can only temporarily monitor the conditions of trachea), the present disclosure has the advantages of monitoring any bleeding, expectoration or position change, and quickly acting in time.

In addition, after the intubation of the endotracheal tube, the patient does not need to be irradiated with chest X-rays in the emergency room or intensive care unit. Through the present disclosure, the position of the endotracheal tube and the relative distance between the endotracheal tube and the carina can be monitored with full-time video monitoring, the depth and position of the endotracheal tube can be adjusted at any time, and the endotracheal tube can be prevented from slipping off the patient's trachea or slipping into the esophagus. The present invention can avoid the labor, time, and risk of medical staff exposure under radiation environment in the chest X-rays operation, and can greatly improve patient's safety.

Moreover, similar to the conventional technology of operating the optical stylet, the optical stylets can enter the tubular body through the primary channel and the secondary channel respectively. The optical stylet that enter the primary channel extends out of the rear end of the tubular body, so that it can be mainly used to guide and explore the trachea and to search for glottis. This is the conventional optical stylet intubation operation. The optical stylet in the secondary channel can continuously monitor the intubation process of the endotracheal tube at all times. As a result, the present disclosure can integrate and maintain the use of conventional optical stylet technology in endotracheal tube intubation, so that the first-time users can use it immediately. The present disclosure further uses the optical stylet in the auxiliary secondary channel to perform video monitoring, so as to achieve and create an intubation technology with a safer and more efficient multi-angle wide-vision video monitoring.

### BRIEF DESCRIPTION OF THE DRAWINGS

The present invention will be further described in detail below in conjunction with the drawings and specific embodiments.
FIG. 1 is a schematic diagram showing the appearance of a first embodiment of this disclosure;
FIG. 2 is an exploded view of the first embodiment of this disclosure;
FIG. 3 is a sectional view of FIG. 2 along the line 3-3;
FIG. 4 is a side view of a first aspect of the first embodiment of this disclosure, wherein the oblique angle of the rear end of the tubular body is 35 degrees;
FIG. 5 is a schematic diagram showing the operation (first aspect) of using the optical stylet to capture video;
FIG. 6 is a side view of a second aspect of the first embodiment of this disclosure, wherein the oblique angle of the rear end of the tubular body is 50 degrees;
FIG. 7 is a schematic diagram showing the operation (second aspect) of using the optical stylet to capture video;
FIG. 8 is a schematic diagram showing the operation of the endotracheal tube of this disclosure;
FIG. 9 is a schematic diagram showing the appearance of a second embodiment of this disclosure.

### DETAILED DESCRIPTION OF THE DISCLOSURE

The following describes the technical solutions in the embodiments of the present invention clearly and completely in conjunction with the drawings in the embodiments of the present invention. In the following description, many specific details are explained in order to fully understand the present invention, but the present invention can also be implemented in other ways different from those described here, and those skilled in the art can do similar modification without departing from the connotation of the present invention. Therefore, the present invention is not limited by the specific embodiments disclosed below.

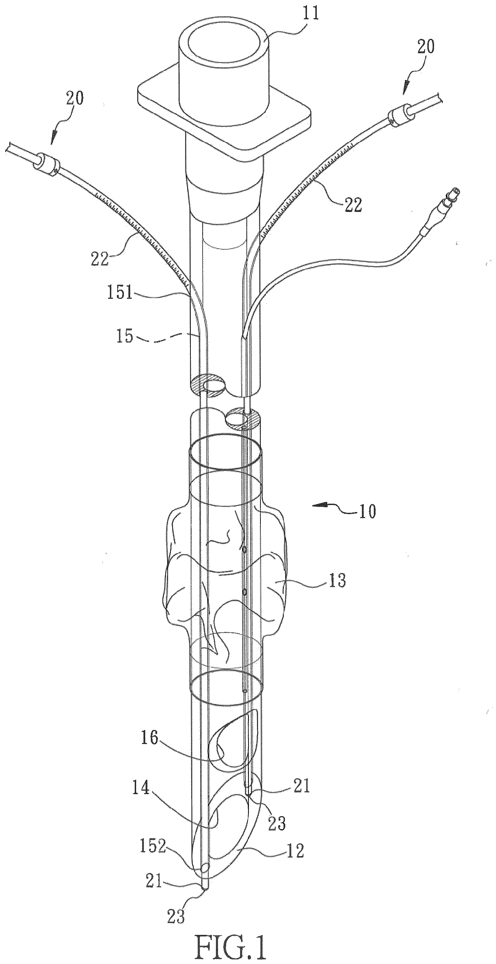

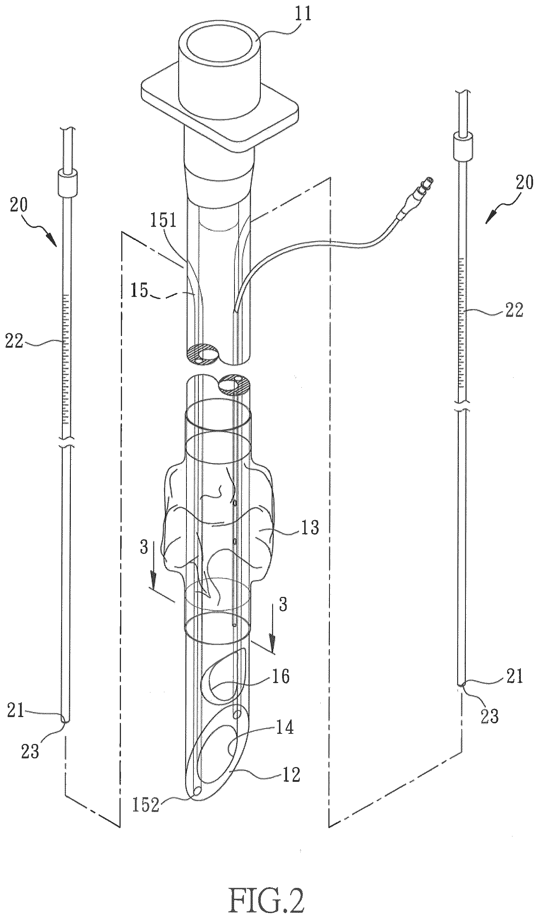

Referring to FIGS. 1 to 9, this disclosure provides an endotracheal tube with built-in video endoscope function for full-time video monitoring, which comprises:

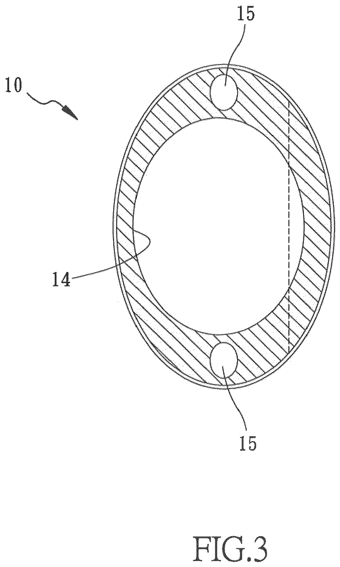

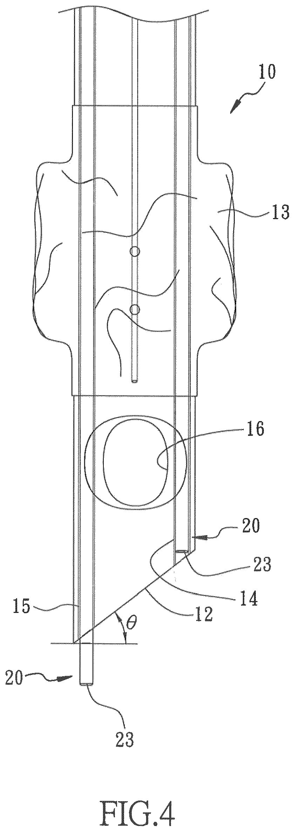

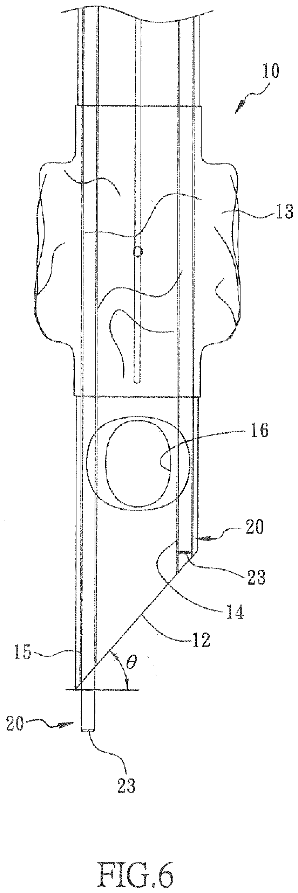

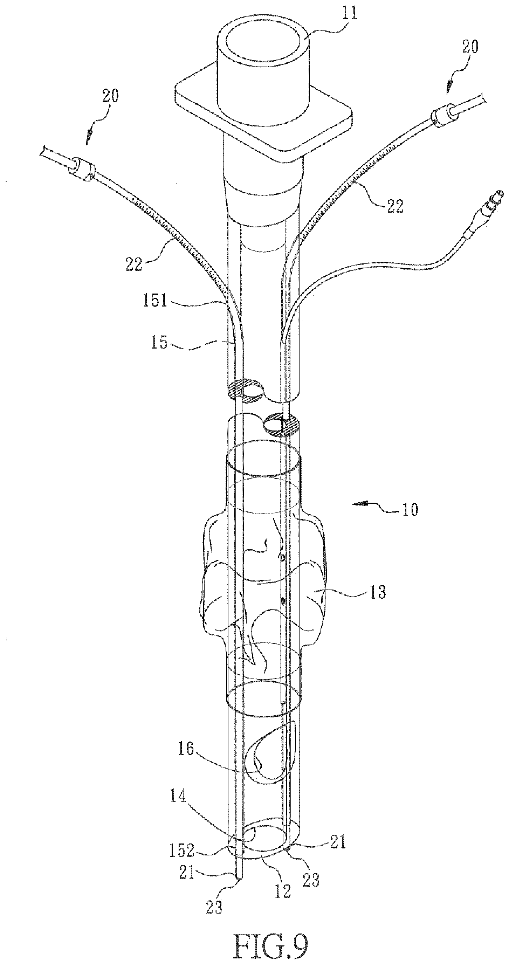

A tubular body 10, which is flexible and has a pre-shaped curvature, wherein the radial cross-section of the tubular body 10 is substantially elliptical. As shown in FIG. 3, the tubular body 10 is to be placed in a trachea 2 of a patient 1. The tubular body 10 has a front end 11 and a rear end 12 opposite to each other. The front end 11 is connected to a ventilator, and an inflatable bag 13 is provided close to the rear end 12. Herein, the rear end 12 of the tubular body 10 has an oblique opening structure or a flat opening structure. In a first embodiment of this disclosure, the rear end 12 of the tubular body 10 has an oblique opening structure, and an oblique angle θ of the rear end 12 of the tubular body 10 is greater than 0 and less than 90 degrees. As shown in FIG. 4, the oblique angle θ of the rear end 12 is 35 degrees. As shown in FIG. 6, the oblique angle θ of the rear end 12 is 50 degrees. A larger oblique angle θ indicates a larger oblique surface of the rear end 12. Accordingly, during the operation of placing the tubular body 10 in the trachea 2 of the patient 1, when the rear end 12 is in contact with the protruding features of the airway (e.g. vocal cords and nasal concha), the endotracheal tube with the flexible tubular body 10 and the oblique opening of the rear end 12 can be more smoothly and gently curved around the protruding features, thereby effectively avoid the trauma of the protruding features. In the second embodiment of this disclosure, the rear end 12 of the tubular body 10 has a flat opening structure as shown in FIG. 9.

Moreover, a primary channel 14 runs through the tubular body 10. The primary channel 14 runs through the front end 11 and the rear end 12 of the tubular body 10, so that the ventilator supplies oxygen to the trachea 2 of the patient 1 through the primary channel 14. As shown in FIG. 3, the radial cross-section of the primary channel 14 is substantially elliptical.

A secondary channel 15 also runs through the tubular body 10. The secondary channel 15 is located next to the primary channel 14, and is provided with a front opening 151 and a rear opening 152 communicating with each other. The front opening 151 is located at a side edge of the tubular body 10, and the rear opening 152 is arranged at the rear end 12. When the tubular body 10 is placed in the trachea 2 of the patient 1, the front opening 151 is positioned higher than a bite part 3 of the patient 1. In particular, the front opening 151 is located in front of the bite part 3; that is the front opening 151 is exposed out of the bite part 3 and is visible. In this disclosure, a plurality of the secondary channels 15 can be provided, and the secondary channels 15 are arranged with equivalent spaced angle. In this embodiment, as shown in FIG. 3, two secondary channels 15 are provided, and the two secondary channels 15 are arranged with equivalent spaced angle of 180 degrees.

In addition, the side edge of the tubular body 10 is formed with a Murphy eye 16, and the Murphy eye 16 is close to the rear end 12 and communicates with the primary channel 14. Herein, the aperture size of the Murry eye 16 is equal to an inner diameter of the primary channel 14, or the aperture size of the Murry eye 16 is smaller than the inner diameter of the primary channel 14 and larger than an inner diameter of the secondary channel 15. In this embodiment, the aperture size of the Murry eye 16 is equal to three quarters of the inner diameter of the primary channel 14.

An optical stylet 20, which has a length greater than that of the tubular body 10 and is flexible. The curvature of the optical stylet 20 can fit the curvature of the tubular body 10. The optical stylet 20 runs through the front opening 151 and the rear opening 152 of the secondary channel 15. When the end 21 of the optical stylet 20 is located at the rear opening 152, a part of the optical stylet 20 coming out from the front opening 151 has a measuring scale 22. In this embodiment, the amount of the optical stylets 20 matches the amount of the secondary channels 15, and each optical stylet 20 runs through one corresponding secondary channel 15.

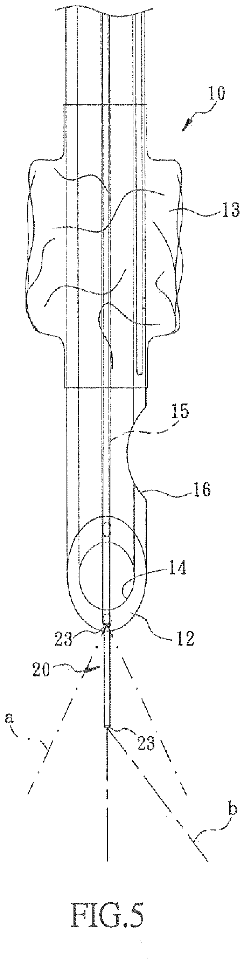

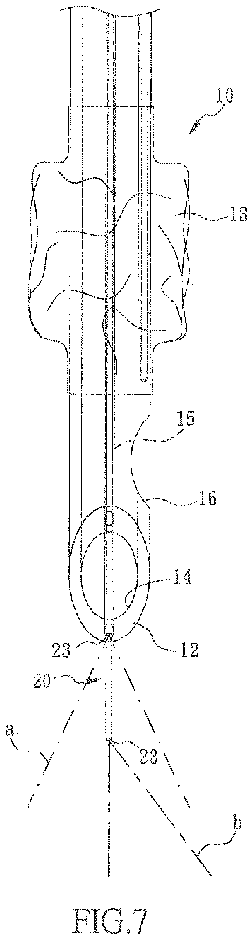

The end 21 of the optical stylet 20 is provided with a camera device 23, and the camera device 23 comes out from the rear opening 152. The camera device 23 is configured to capture the video inside the trachea 2 of the patient 1. Each optical stylet 20 comes out from the rear opening 152 of the corresponding secondary channel 15, and the camera devices 23 aim to different directions to capture the video. With reference to FIGS. 5 and 7, the camera devices 23 of two optical stylets 20 aim to the direction a and the direction b, respectively, to capture the videos. Accordingly, the captured videos can compensate each other and are partially overlapped. This configuration can utilize the multiple angles and wider visions to assist in finding and positioning the glottis, and helping to quickly and effectively perform intubation of the trachea 2.

In addition, the camera device 23 is coupled to a display. The display can be an eyepiece or a remote screen, and the display can be coupled to the camera device 23 by wired connection or wireless connection. When the display is an eyepiece, the display is disposed at another end of the optical stylet 20 other than the camera device 23 and is coupled to the camera device 23 by wired connection. When the display is a remote screen, the display is coupled to the camera device 23 by wireless connection.

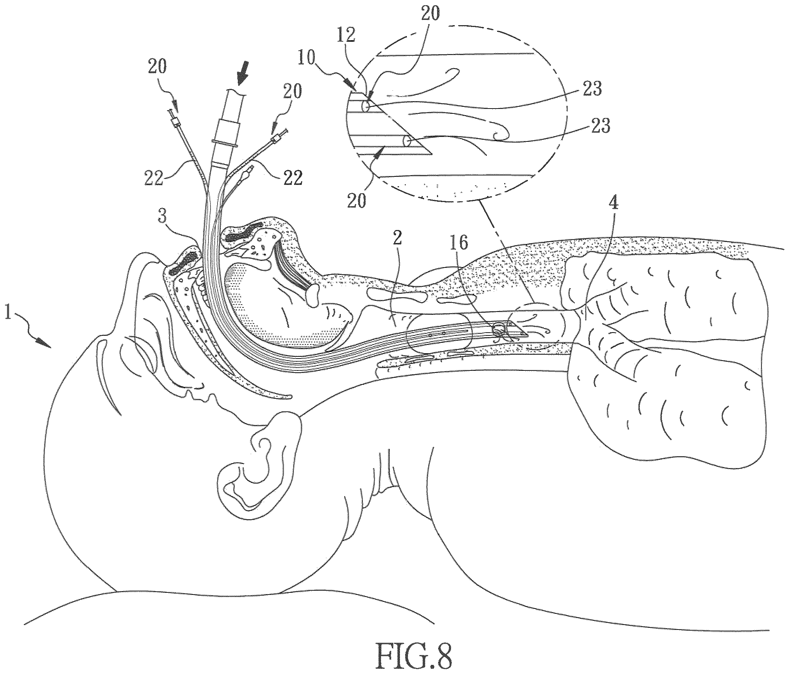

As shown in FIG. 8, the operator inserts the optical stylet 20 into the secondary channel 15, and the secondary channel 15 comes out from the rear end 12 of the tubular body 10 so as to serve as a guide for finding and positioning the glottis. First, the optical stylet 20 is put into the trachea 2 of the patient 1. At this time, the camera device 23 at the end 21 of the optical stylet 20 can capture the image of the trachea 2. After the operator sees the carina 4 of the patient 1 through the display, and confirms that the optical stylet 20 is located in the trachea 2 of the patient 1, the tubular body 10 is moved along the optical stylet 20 and then placed in the trachea 2 of the patient 1. This method is called railroad introduction to ensure the position of the tubular body 10 in this intubation. At this time, the optical stylet 20 as a guide is pulled out and retrieved, so that the end 21 of the optical stylet 20 is parallel to the tubular body 10 or slightly pulled back and retracted, and then it can be used as an optical stylet 20 for full-time monitoring.

Then, through the measuring scale 22 of the optical stylet 20, the length of the extending or retracting part of the optical stylet 20 can be checked at any time. After the bite portion 3 of the patient 1 bites the tubular body 10, the procedure of inserting the tubular body 10 into the trachea 2 (intubation) is completed. Afterwards, the operator can connect the tubular body 10 to a ventilator, so that the ventilator can deliver oxygen through the primary channel 14 to the trachea 2 of the patient 1.

The secondary channels 15 are located at two opposite sides of the tubular body 10, so that the operator can, based on the habits of the dominant hand, penetrate the optical stylet 20 that is mainly manipulated into one of the secondary channels 15 corresponding to the dominant hand. Another optical stylet 20 running through the other secondary channel 15 is used to enlarge the camera vision, thereby utilizing the multiple angles and wider visions to assist in finding and positioning the glottis, and helping to quickly and effectively perform intubation of the trachea 2. As shown in FIG. 5, the two camera devices 23 of two optical stylets 20 aim in the direction a and the direction b, respectively.

Accordingly, since the oxygen supplied from the ventilator and the optical stylet 20 can enter the tubular body 10 through the primary channel 14 and the secondary channel 15, respectively, the optical stylet 20 does not need to be pulled out during the entire intubation process of the trachea 2. The operator can continuously monitor the intubation process at all times through the camera 23 at the end 21 and the display. This configuration can achieve the function of full-time monitoring the position of the endotracheal tube, which can effectively prevent the intubation position from slipping and mistakes.

During the intubation process of the endotracheal tube 2, the part of the optical stylet 20 protruding from the rear end 12 of the tubular body 10 is about eight to ten centimeters as a guide. Through the camera devices 23 at the ends 21 of the optical stylets 21 and the display coupled to the camera devices 23, the operator can continuously monitor the endotracheal intubation process of the endotracheal tube at all times and use the rear end 12 of the tubular body 10 to identify and find the entrance of the trachea 2 and the glottis. Different from the intubation process with the conventional stylet, the optical stylet 20 of the present disclosure is pulled back and retracted to be even with the rear end 12 of the tubular body 10 after completing the intubation of the endotracheal 2. At this time, the stylet 20 can function as a full-time monitor for monitoring the internal condition of trachea 2, bleeding or expectoration, and the position of the endotracheal tube 2. Compared to the conventional optical stylet, which must be removed after the intubation of trachea 2 (so it can only temporarily monitor the conditions inside trachea), the present disclosure has the advantages of monitoring any bleeding, expectoration or position change, and quickly acting in time.

In addition, after the intubation, the patient 1 does not need to be irradiated with chest X-rays in the emergency room or intensive care unit to check the position of the endotracheal tube 2. Through the camera device 23 of the optical stylet 20 and the display, the position of the tubular body 10 and the relative distance between the rear end 12 and the carina can be monitored with full-time video monitoring, and the depth and position of the tubular body 10 can be adjusted at any time, thereby preventing the endotracheal tube from slipping off the patient's trachea 2 or slipping into the esophagus. As a result, the present invention can avoid the labor, time, and risk of medical staff exposure under radiation environment in the chest X-rays operation, and can greatly improve patient's safety.

In addition, the radial cross-section of the tubular body 10 is substantially elliptical, which can fit the mouth shape of the patient 1, so that the patient 1 can easily bite the tubular body 10 without opening the mouth too much, thereby providing the patient 1 with comfort during biting.

In addition, similar to the conventional technology of operating the optical stylet 20, the optical stylets 20 can enter the tubular body 10 through the primary channel 14 and the secondary channel 15 respectively. The optical stylet 20 that enter the primary channel 14 extends out of the rear end 12 of the tubular body 10, so that it can be mainly used to guide and explore the trachea 2 and to search for glottis. This is the conventional optical stylet 20 intubation operation. The optical stylet 20 in the secondary channel 15 can continuously monitor the intubation process of the endotracheal tube at all times. As a result, the present disclosure can integrate and maintain the use of conventional optical stylet 20 technology in endotracheal tube intubation, so that the first-time users can use it immediately. The present disclosure further uses the optical stylet 20 in the auxiliary secondary channel 15 to perform video monitoring, so as to achieve and create an intubation technology with a safer and more efficient multi-angle wide-vision video monitoring.

The above description only shows the preferred embodiments of the present invention. Obviously, the described embodiments are only a part of the embodiments of the present invention instead of all the embodiments. Based on the embodiments of the present invention, all other embodiments obtained by those of ordinary skill in the art without creative work shall fall within the scope of the present invention.

## Claims

1. An endotracheal tube with built-in video endoscope function for full-time video monitoring, which is **characterized in**: comprising:
a tubular body being flexible and having a pre-shaped curvature, wherein the tubular body is to be placed in a trachea of a patient, the tubular body has a front end and a rear end opposite to each other, the front end is connected to a ventilator, the tubular body is configured with an inflatable bag, a primary channel and a secondary channel run through the tubular body, the primary channel runs through the front end and the rear end, so that the ventilator supplies oxygen to the trachea of the patient through the primary channel, the secondary channel is located next to the primary channel and is provided with a front opening and a rear opening communicating with each other, the front opening is located at a side edge of the tubular body, the rear opening is arranged at the rear end, and when the tubular body is placed in the trachea of the patient, the front opening is positioned higher than a bite part of the patient and exposed in front of the bite part; and
an optical stylet having a length greater than that of the tubular body and being flexible, wherein the optical stylet runs through the front opening and the I rear opening of the secondary channel, an end of the optical stylet is provided with a camera device, and the camera device is coupled to a display, and the end of the optical stylet comes out from the rear opening to capture a video in the trachea of the patient.

2. The endotracheal tube of claim 1, which is **characterized in**: when the end of the optical stylet is located at the rear opening, a part of the optical stylet coming out from the front opening has a measuring scale.

3. The endotracheal tube of claim 1, which is **characterized in**: the rear end of the tubular body has an oblique opening structure, and an oblique angle of the rear end of the tubular body is greater than 0 and less than 90 degrees.

4. The endotracheal tube of claim 1, which is **characterized in**: comprising a plurality of the secondary channels, wherein the optical stylet runs through one of the secondary channels.

5. The endotracheal tube of claim 4, which is **characterized in**: the secondary channels are arranged with equivalent spaced angle.

6. The endotracheal tube of claim 4, which is **characterized in**: the optical stylet comes out from the rear opening of the corresponding secondary channel, and the camera devices aim to different directions to capture the video.

7. The endotracheal tube of claim 1, which is **characterized in**: the side edge of the tubular body is formed with a Murphy eye, and the Murphy eye is close to the rear end and communicates with the primary channel.

8. The endotracheal tube of claim 7, which is **characterized in**: an aperture size of the Murry eye is equal to an inner diameter of the primary channel, or the aperture size of the Murry eye is smaller than the inner diameter of the primary channel and larger than an inner diameter of the secondary channel;
the endotracheal tube of claim 1, which is **characterized in**: a radial cross-section of the tubular body is substantially elliptical, and a radial cross-section of the primary channel is substantially elliptical.

9. The endotracheal tube of claim 1, which is **characterized in**: the camera device is remotely coupled to the display.
